# EUROPEAN PATENT APPLICATION

(11) **EP 4 635 415 A1**
(43) Date of publication of application: **22.10.2025**
(21) Application number: 24207779.0
(22) Date of filing: 21.10.2024
(51) Int. Cl.: A61B 5/145, B01L 3/00, G01N 27/327, G01N 27/413, G01N 27/416, G01N 27/48

(54) **ELECTROCHEMICAL DEVICE WITH INTEGRATED FLEXIBLE FIBRILLARY STRUCTURE**

(30) Priority: 18.04.2024 RO 202400191
(71) Applicant: Institutul National de Cercetare-Dezvoltare Pentru Fizica Materialelor-INCDFM Bucuresti, Magurele, Jud. Ilfov (RO)
(72) Inventor: BOTTA, Oana-Daciana, Bucuresti (RO); DICULESCU, Victor Constantin, Bucuresti (RO); ENCULESCU, Ionut-Marius, Curtea de Arges, Jud Arges (RO); BEREGOI, Mihaela, Bucuresti (RO); EVANGHELIDIS, Alexandru Ionut, Bucuresti (RO); MATEI, Elena, Magurele, Jud Ilfov (RO)
(74) Representative: Munteanu, Manuela-Cornelia

(57) **Abstract**

The invention relates to an electrochemical device with an integrated flexible fibrillary structure having applicability in performing electrochemical analysis or measurements including, but not limited to, the field of sensors, as well as to a procedure of fabricating such an electrochemical device, and to methods of electrochemically detecting an analyte present in a fluid sample using said electrochemical device.

## Description

### Technical Field

The invention describes an electrochemical device with integrated flexible fibrillary structure, with applicability in performing electrochemical analysis or measurements including, but not limited to, the field of sensors, which can be used with liquid electrolytes.

### Background

Electrochemical techniques involve the study of charge transfer processes driven by redox reactions taking place at the interface between the working electrode and the electrolyte medium, therefore the optimal contact between the two is of utmost importance. Portable electrochemical cells enable quantitative and qualitative analysis on samples in real time, outside a specialized laboratory, in order to identify and quantify chemical compounds such as: toxic contaminants, ions or molecules involved in physiological processes, genetic material of viral origin or biomarkers associated with chronic diseases (e.g. cancer, diabetes, hepatitis, etc.).

The current interest in the development of fast-response sensors with high portability, sensitivity and specificity has led to the fabrication of electrochemical cells on porous substrates, fibrillary structures being generally preferred. A fibrillar porous material is any structure composed of single fibers, derived from any type of material, whose spatial arrangement results in the formation of voids called pores. The main motivation for using porous materials as substrates is given by the possibility of passively driving fluid flow under the action of capillary force. It is worth noting that in this type of flow, mass transport (the movement of the fluid and the molecules dissolved in it) is carried out under the action of forces such as electrostatic interactions or pressure or concentration gradients. Thus, this process is strongly influenced by the structure of the porous medium and the chemical structure of the surface. In the fabrication of current portable electrochemical cells there is a clear preference for choosing paper as substrate, which may or may not be provided with fluidic channels. These fluidic channels are hydrophilic channels created in paper, either by wax-printing, laser-cut printing, or other microfabrication techniques. The purpose of fluidic channels is to direct liquids towards specific regions of the paper, facilitating the chemical reactions necessary for electrochemical measurements.

Current designing techniques employ planar architectures in which the electrodes are fabricated directly on the paper surface, or multilayered architectures in which the electrodes are fabricated on additional layers and are brought into contact with the fluid-containing area either by mechanical pressing or by using adhesives. The methods generally used to fabricate electrodes on porous substrates include deposition techniques of materials such as: graphite layers by pencil drawing; conductive inks by screen-printing or pen-plotting; laser-induced graphene; thin metal layers by physical vapor deposition techniques.

A first limitation of these electrochemical devices fabricated by the aforementioned manufacturing techniques is represented by the lack of mechanical strength, due to the properties of the deposited materials, which are prone to cracking under the action of mechanical forces, especially in the case of bending movements, implicitly causing electrical conduction interruption.

In addition, in the case of planar architectures using paper as a substrate, the deposition of any material directly on the paper surface will induce physical pore blockages or chemical surface changes, restricting fluid flow by capillary action. This is detrimental to sensing applications, as flow restriction limits the contact area between the electrode and the fluid, reducing charge transfer processes and hence the electrochemical response.

In the case of multilayered architectures, the disadvantage of depositing materials on different substrates involves the need for subsequently bringing the substrate containing the conductive material into contact with the substrate containing the fluid, either by mechanical pressing or by using adhesives. The disadvantage of such an approach is that it does not ensure an optimal and reproducible contact between the electrodes and the fluid and leads to an increase in the charge transfer resistance between the electrode and the electrolyte.

The fabrication of electrodes for electrochemical devices involves precise processes of synthesis and integration of active materials on suitable supports. The electrodes must provide high surface area, conductivity and mechanical stability.

Fabricating the electrodes by depositing materials such as conductive inks directly on the porous substrates is not ideal. Conductive inks are generally made from carbon-based materials (graphite, carbon black) dissolved in organic solvents, which may not be compatible with porous substrates and may reduce electrode efficiency and performance. Any solvent in contact with the porous medium will diffuse into it, and in the case of organic solvents this leads to a contamination of the medium and changes in chemical structure, leading to reduced biocompatibility.

Moreover, current commercial devices as well as most of those presented in the scientific literature are manufactured with electrodes placed in the same plane. This may cause an uneven charge distribution at the working electrode surface.

The technical problem that the invention solves is represented by: the low mechanical strength of the electrodes, the contamination of the porous hydrophilic fibrillary substrate, the physical blockage of the pores due to the electrode manufacturing process, and the imperfect contact between the electrodes and the substrate containing the fluid.

The present invention overcomes the problems identified in the state-of-the-art by providing an electrochemical device for detecting analytes comprising polymeric layers made of polymeric fiber meshes and a fibrillar hydrophilic substrate disposed between the two polymeric layers, a method for fabricating the electrochemical device, and a method for electrochemically detecting an analyte present in a fluid sample using the electrochemical device.

### Summary of the Invention

The invention discloses an electrochemical device, for detecting an analyte, comprising:
- a first polymeric layer, comprising a first electrode,
- a second polymeric layer comprising a second electrode,
- a third layer for the placement of an analyte, located between the two polymeric layers, wherein the first and second polymeric layers are made of polymeric meshes, formed of polymeric fibers, and
wherein the third layer comprises a fibrillar hydrophilic substrate having at least one hydrophilic channel, comprising an analyte insertion zone and at least one hydrophilic region delimited by a fluid impermeable barrier, and wherein the hydrophilic region is in contact with the two electrodes.

The novelty of this invention consists in the use of submicrometer polymeric fibers as substrate for electrode fabrication, on which the conductive material is deposited, integrated with a fibrillar hydrophilic substrate. The electrochemical device is fully integrated by heat transfer of the conductive polymeric fiber mesh onto the paper substrate. This configuration has significant advantages such as: the large contact area provided by the geometry of the fibers, the flexibility of the whole assembly and the maintenance of the structural integrity of the electrodes and the paper. The polymeric fibres have diameters ranging from 100 - 900 nm, preferably 200 - 500 nm, most preferably 300 nm, preferably with an optical transmittance of 25% ± 2%. The metallic layers have a thickness between 5-300 nm, preferably 100-250, most preferably 100 nm and are deposited on the surface of the fibers.

The electrochemical device, proposed by the present invention, employs electrodes with a flexible fibrillar structure obtained from conductive polymeric fibers, placed on the surface of a hydrophilic substrate with fibrillar structure (paper, textile or any other polymeric substrate), which has a role both in directing the flow and in storing the fluids used in the analysis, as well as redox probes or other macromolecules with a role in biorecognition (such as nucleic acids, enzymes, antibodies, etc.).

Conductive polymeric fibers can be fabricated from solutions of: poly(3,4-ethylenedioxythiophene) (PEDOT), poly(3,4-ethylenedioxythiophene) polystyrene sulfonate (PEDOT: PSS), polyaniline (PANI), polypyrrole (PPy), poly(methyl methacrylate) (PMMA), nylon, polycaprolactone (PCL), poly(lactic acid) (PLA), cellulose acetate (CA), polyethylene oxide (PEO), polyvinyl chloride (PVC), polyurethane (PU), polyvinylidene fluoride (PVDF) or combinations thereof.

The electrochemical device with integrated flexible fibrillary structure can be developed in a multiple electrode system, arranged on each side of the fibrillar hydrophilic substrate.

The first advantage of the proposed invention is the flexibility of the conductive polymeric fibers, which have mechanical resistance to bending and twisting motions without suffering structural defects.

The electrodes made of conductive polymeric fiber meshes, namely the working electrode, the auxiliary electrode and the reference electrode, have the advantages of flexibility and superior mechanical bending strength, as well as a large contact area.

The arrangement of the electrodes on both sides of the paper is also an advantage. The placement of the auxiliary electrode below the working electrode, separated by the paper, results in a uniform electric field distribution between the electrodes. This arrangement has been optimized and supported by experimental data.

The materials used for the electrodes can be gold, platinum, palladium, silver or carbon. The choice of material is not restricted by any limitation as long as charge transfer can take place between the conductive material and the chemical species of interest. Noble metals such as gold, platinum and palladium are preferred for the manufacture of both working and auxiliary electrodes because of their chemical stability, which allows for a long storage life of the electrochemical device from the time of manufacture to the time of measurement. Moreover, these metals present specific advantages: gold offers superior flexibility; gold-coated polymeric fiber meshes have high mechanical resistance to bending and twisting; platinum and palladium have the ability to incorporate hydrogen ions into their structure and are useful in the electrochemical detection of H₂O₂ at low potentials (± 100 mV).

Although all the electrodes can be fabricated from the same material, it is preferred that the reference electrode be fabricated from silver / silver chloride due to the stability of the standard electrode potential.

The electrochemical device may comprise on one of the polymer layers a zone for the immobilization of a reagent, located on the electrode surface, in the area that is in contact with the hydrophilic region.

Moreover, the electrochemical device may comprise at least one zone for immobilizing a reagent located on the third fibrillar hydrophilic layer, located between the two polymeric layers.

This fibrillar hydrophilic layer, made of paper or textile, allows for the storage of the reagents of interest and the analysis fluid, and also allows for the passive flow of fluid under the action of the capillary force.

The hydrophilic channel offers advantages such as precise handling of very small volumes of fluid, low reagent and sample consumption, and the possibility of integration into complex systems for automating analytical and experimental processes.

The hydrophobic barrier borders the hydrophilic channel, keeping the assay fluid in the vicinity of the electrodes and allowing the clear definition of the contact area between the electrodes and the fluid, thus establishing the analysis region.

Placement on or integration with a flexible fibrillar substrate, such as paper or textiles, results in a superior mechanical strength of the polymeric fibers. The fibrillar hydrophilic substrate is flexible and can be made of cellulose paper, cotton paper, glass fiber paper, filter paper, chromatography paper, commercial printing or writing paper, cotton fabric, polyester fabric, polyamide fabric, polyacrylamide hydrogels.

The use of paper as an electrode support is advantageous because it has a good mechanical strength and electrolyte absorption capacity, providing efficient ion diffusion, ensuring durability and optimal performance of the electrochemical device.

The conductivity of the polymeric fibers that compose the polymeric meshes used to obtain the electrodes with flexible fibrillary structure, can be ensured either by depositing thin conductive layers on their surface or by incorporating conductive materials directly into the fiber structure. Metallic particles of silver, copper, gold, nickel, aluminium, platinum or semiconductor particles of titanium dioxide, zinc oxide, cadmium selenide, cadmium sulphide, gallium arsenide, zinc selenide may be used. The deposition of the particles on the surface of the polymeric fibers has the advantage that it leads to the formation of a thin layer, which improves the conductivity of the electrodes. The incorporation of conductive or semiconducting particles, such as nanoparticles (Au, Ni, Pt, C in the form of carbon black or fullerene), but also 1D materials such as carbon nanotubes or 2D materials such as graphene, offers the advantage of integrating these particles into the structure of the polymeric fibers during the manufacturing process, which gives them conductive properties without modifying the outer surface of the fibers.

In this way, the electrodes can be obtained as a unitary system, eliminating the possibility of substrate contamination during the fabrication process. Through the use of masks in the metallization process, alternating conductive / insulating regions can be formed, thereby leading to the possibility of forming multiple electrodes on the surface of a conductive polymeric fiber mesh, which contributes to improved performance and functionality of the electrochemical device.

The invention further discloses a method for fabricating an electrochemical device according to claim 1. The fabrication method comprises three main steps: fabrication of the fluidic channel, fabrication of the electrodes, and integration of these components.

Step 1 of fabricating a hydrophilic channel in a fibrillar hydrophilic substrate, involves i. providing the fibrillar hydrophilic substrate; ii. designing the geometry of the hydrophilic channel; iii. printing the hydrophilic channel onto the hydrophilic substrate, by depositing a fluid impermeable layer on the hydrophilic substrate in order to delimit a hydrophilic region; iv. heat treating the fibrillar hydrophilic substrate onto which the hydrophilic channel has been imprinted.

Step 2 of fabricating electrodes on a first polymeric layer and a second polymeric layer, involves: i. fabricating the polymeric fibers by spinning a polymer solution; ii. collecting the polymeric fibers from the preceding step on a conductive frame in order to obtain a polymeric mesh; iii. measuring the optical transmittance of the polymeric meshes by UV-Vis spectrophotometry; iv. fabricating the electrodes by metallizing at least a part of the surface of each polymeric fiber mesh,

Step 3 of assembling the electrochemical device involves placing the fibrillar hydrophilic substrate containing the hydrophilic channel, resulting from step 1, between the first and second polymeric layers, comprising the first and second electrodes, respectively, resulting from step 2, and heating the whole assembly at temperatures between 50 and 150 °C for 2-15 minutes.

In the fluidic channel fabrication step, the design of the channel geometry may be carried out in a CAD program.

The printing of the geometry may be accomplished on paper or textiles using a 3D printer and wax or polymeric filaments with low melting points.

The heat treatment of the hydrophilic substrate in step 1 can be performed at 185°C for 20 minutes and has the advantage of creating the impermeable barrier in the full thickness of the substrate, by melting the printed layer, physically blocking the pores and fully integrating it into the hydrophilic substrate.

This step ensures fluid confinement in a well-defined area, subsequently defining the active surface area of the working electrode, as well as allowing the operation with small volumes of fluid, on the order of microliters.

By measuring the optical transmittance of the polymeric fiber meshes the reproducibility of the electrodes can be ensured in terms of: fiber density, which subsequently defines the effective contact area with the fluid; and current density. Reproducibility of the electrode characteristics can be obtained by using only polymeric fiber meshes with the same transmittance value. Moreover, selective metallization using deposition masks allows the fabrication of multiple electrodes on a single polymeric fiber mesh, ensuring reproducibility in terms of architecture and electrical characteristics.

The assembly procedure of the electrochemical cell includes:
1. Preheating an oven to 100 °C for 10 minutes;
2. Placing the paper containing the fluidic channel on a glass support;
3. Placing the polymeric fiber mesh on the paper surface;
4. Placing the assembly in the oven and performing the heat treatment at temperatures between 50 and 150 °C for 2-15 minutes;
5. Removing the assembly from the oven, detaching the metal frame, which was supporting the polymer fiber meshes and repeating the procedure on the reverse side.

The preheating step of the oven ensures uniform heat distribution within its enclosure and subsequently around the assembly, resulting in a uniform heat treatment across the entire surface of the assembly. The use of the glass support facilitates the handling of the assembly, protects the paper from the metal elements of the oven, which could cause uneven heating and burning of the paper, and allows easy removal of the metal frame. The gravitational force combined with the heating action determine the detachment of the metal frame from the polymeric fiber meshes, the latter remaining on the paper. The placement of the polymeric fiber meshes on both sides of the paper offers specific advantages: by placing the reference electrode on the same side and in the vicinity of the working electrode a finer control over the applied potential can be ensured, and placing the auxiliary electrode on the underside, below the working electrode, ensures a uniform electric field distribution.

Polymeric fibers can be fabricated under the action of a different range of forces which include: electrostatic (electrospinning), centrifugal (rotary-jet spinning) or gravitational (dry spinning) forces. Electrospinning allows for the fabrication of polymeric fiber meshes with a high degree of uniformity or even directionality, by fine tuning environmental and process parameters. The polymeric fibers formed by electrospinning can reach diameters in the micro- and nanometer range. As consequence, electrodes fabricated from polymeric meshes will have higher contact surface areas and sensitivities compared to planar electrodes.

The main advantages of the electrochemical device configuration that integrates these polymeric meshes consist in: high surface area determined by the fibers geometry, the flexibility of the whole assembly, as well as the high degree of structural integrity of both the electrodes fabricated from the polymeric fiber meshes and the fibrillar hydrophilic substrate and the fluidic channel printed on it.

Selective metallization leads to the formation of alternating conductive and insulating zones, the latter facilitating the attachment of the polymeric meshes on the porous hydrophilic substrates with fibrillar structure. Through a heat treatment procedure, adhesion between the uncoated polymeric fibers and the substrate can be induced. However, the metal-coated polymeric fibers will retain their structural integrity, as the metallic layer has a higher melting point than the uncoated polymeric fibers. In this manner, an optimal contact between the metallized polymeric fibers and the hydrophilic substrate fibers can be achieved without the use of adhesives.

The attachment of the metallized polymeric fibers on both sides of the paper was made possible by optimizing the heat treatment procedure in terms of time, temperature and uniform heat distribution, as well as optimizing the metal deposition procedure so that the coated fibers retain their flexibility.

A further advantage of the device according to the invention is its structural arrangement, as the placement of the auxiliary electrode below the working electrode results in a uniform electric field distribution.

The electrochemical device proposed by the present invention can be used for measurements that include voltammetric, amperometric, potentiometric, impedimetric or conductimetric techniques.

The invention further discloses an electrochemical detection method for an analyte present in a fluidic sample using the electrochemical device according to claim 1, comprising the steps of: a. depositing on one of the layers a solution containing a reagent; b. depositing the fluidic sample containing the analyte in the hydrophilic channel of the fibrillar hydrophilic substrate until a fluidic contact between the sample, the reagent and the electrodes is achieved; and c. measuring an electrochemical signal using the electrodes.

The electrochemical device proposed by the present invention has the advantage of being reusable as long as it is used for the same type of application, which means analysis of the same molecular species with the same type of reagent.

Impregnating the fibrillar hydrophilic substrate with a solution containing a reagent involves:
applying to the desired substrate a solution containing the specific reagent and other ligand molecules or molecules involved in maintaining the stability/conformation of the specific reagent.
The following combinations are possible:
   Specific reagent and ligand molecules involved in controlling the conformation of the specific reagent.
   Examples of specific reagents are: proteins, nucleic acids, organic molecules, metal complexes, salts, acids, bases.
   Examples of ligand molecules involved in controlling the conformation of the specific reagent are: bovine serum albumin, sodium lauryl sulphate, glutaraldehyde, chitosan, alginate, polyethylene glycol, dextran.

Also, the solution containing the specific reagent may be deposited on the surface of the electrodes, subsequent to the formation on their surface of a layer that plays a role in the covalent immobilization of the reagent. Thus, the covalent immobilization can be performed in three steps:
1. Formation of a self-assembled monolayer of alkanethiol molecules with carboxylic or amino terminal functional groups.
2. Activation of the functional groups with (N-hydroxysuccinimide (NHS), 1-ethyl-3-(3-dimethylaminopropyl)-carbodiimide (EDC), carbohydrazide, hydrazine and cyanoborohydrides).
3. Formation of covalent bonds between the specific reagent and the activated terminal functional groups.

The three steps can be accomplished either by immersing the electrodes in the required solutions or by drop-casting each solution on their surface.

### Brief Description of the Drawings

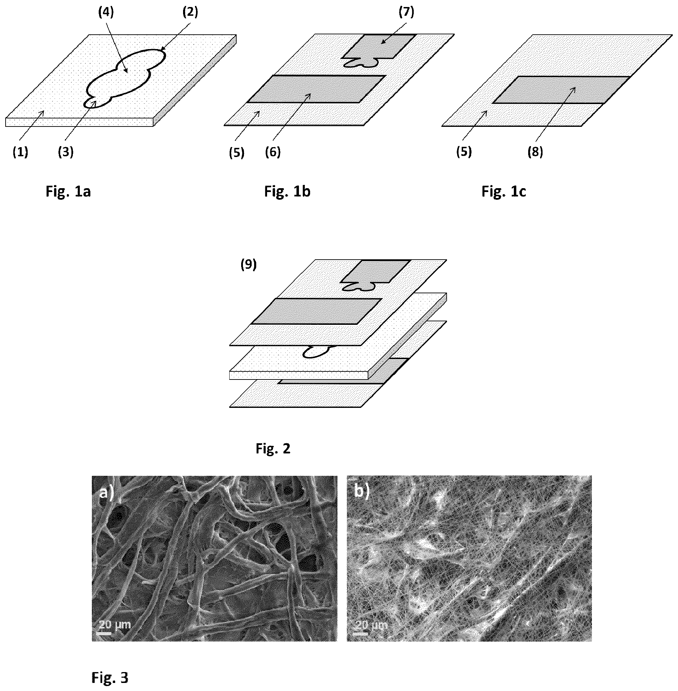

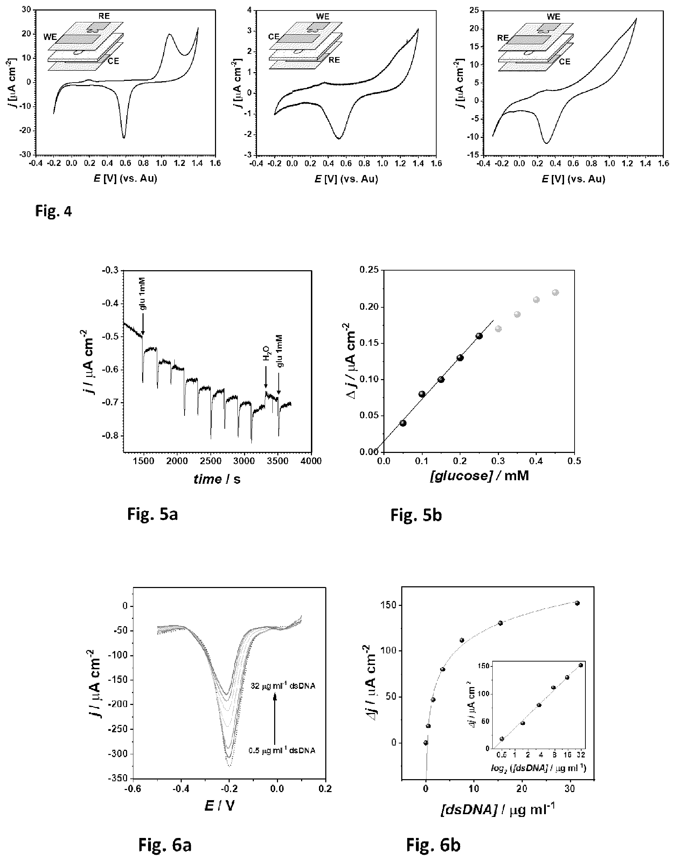

The steps related to a fabrication method of the proposed invention and examples of applications are presented in connection with Figures 1-6, which represent:
- Fig. 1. An example of a possible geometry for the electrochemical device components.
- Fig. 2. An example of an architecture of the electrochemical device 9, presented in exploded view.
- Fig. 3. Electron microscopy images showing the paper before and after the thermal attachment of the metallized polymeric fiber meshes.
- Fig. 4. Cyclic voltammograms recorded in buffer solution performed with the device in configurations with different electrode placement.
- Fig. 5. The graph obtained from the detection of glucose in artificial sweat using the electrochemical device by chronoamperometry at a fixed potential of -0.2 V (vs. Ag), and the linear dependence between the H₂O₂ reduction current and glucose concentration.
- Fig. 6. The graph obtained from the detection and quantification of nucleic acids in the presence of methylene blue in the electrochemical device by differential pulse voltammetry, and the dependence of the reduction current of methylene blue as a function of the concentration of double stranded nucleic acids.

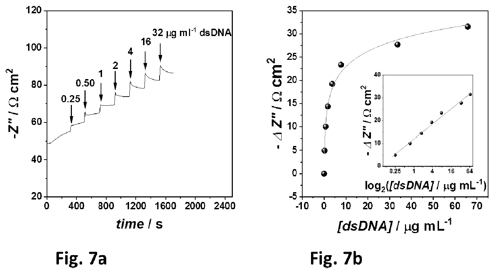

- Fig. 7. The graph obtained from the detection and quantification of nucleic acids using the electrochemical device by electrochemical impedance spectroscopy at open-circuit potential, and the dependence between the imaginary component of the impedance and the concentration of double stranded nucleic acids.

Figure references: (1) hydrophilic substrate, (2) fluid impermeable barrier, (3) fluid insertion zone, (4) analysis zone, (5) polymeric fiber mesh, (6) working electrode, (7) reference electrode, (8) auxiliary electrode, (9) assembled device configuration.

### Examples

**Example 1.** The fabrication method of the electrochemical device comprises the steps:
Step 1. The fabrication of the hydrophilic channel, comprising the steps:
   i. Designing the geometry of the hydrophilic channel;
   ii. Printing the hydrophilic channel on paper by depositing a layer of fluid impermeable material on the hydrophilic substrate so as to outline a hydrophilic region,
   iii. Heat treating the hydrophilic substrate on which the hydrophilic channel has been printed,
Step 2. Electrode fabrication, comprising the steps:
   i. Electrospinning a solution of 10% (w/v) poly(methyl methacrylate) in N,N-dimethylformamide by applying an electrical potential difference of 15 kV between the spinneret and the collector, using a flow rate of 0.5 ml/hr, at a temperature of 23-25 °C and a relative humidity between 22-25 %, the fibers being collected on stainless steel frames of 3 x 3 cm;
   ii. Measuring the optical transmittance of the polymeric fiber meshes by UV-Vis spectrophotometry;
   iii. CAD design of the electrode geometry;
   iv. Fabrication of deposition masks according to the desired geometry;
   v. Covering the polymeric fiber mesh with masks, which allow metal deposition only in predefined regions;
   vi. Metallizing the polymeric fibers by magnetron sputtering with a 100 nm layer, monitored with a quartz crystal microbalance, using a target of the metal of interest (gold, silver, platinum, palladium, etc.) depending on the application.
Step 3. Assembling the electrochemical device by placing the metallized fiber meshes on the surface of the paper containing the fluidic channel and heating the whole assembly at 100 °C for 10 minutes.

Printing the fluidic channel onto the paper can be done using a 3D printer and wax or polymeric filaments with low melting points, followed by heat treatment at 185 °C for 20 minutes to determine the printed layer to melt and penetrate the full depth of the paper to physically block the pores.

**Example 2.** An example of a sensor with optimal functionality was fabricated using a 0.20 mm thick Whatman CHR1 chromatography paper as hydrophilic substrate, on which a barrier with a width of 0.80 mm and a layer height of 0.24 mm was printed using Moldlay filaments. The channel comprised a 4.20 mm diameter insertion zone connected to an 11.00 mm diameter analysis zone and an 8.20 mm diameter zone for the reference electrode. The total area of the realized hydrophilic channel was 1.61 cm². The electrodes were made of poly(methyl methacrylate) meshes, composed of fibers with diameters ranging from (200 - 400) nm, with an optical transmittance of 25% ± 2%. The contact area between the working electrode and the analysis zone was 0.64 cm². Metal layers with a thickness of 100 nm were deposited on the surface of the meshes, using gold for the working and auxiliary electrodes and silver for the reference electrode.

**Example 3.** An example for the determination of the calibration curve for glucose detection in artificial sweat using the described electrochemical device in which the enzyme glucose oxidase has been immobilized is presented below. In the first step, a volume of 40 µL of a 1 % (w/v) solution of glucose oxidase in ultrapure water is introduced into the fluidic channel and allowed to dry for 60 minutes. The modified sensor can be used immediately or stored at +4 °C. For measurements, a volume of 40 µL of artificial sweat is injected into the channel and a constant potential of -0,2 V (vs. Ag) is applied, recording the evolution of current over time. After the current reaches a stable value, volumes of 2 µL of a 1 mM glucose solution are periodically injected into the channel every 200 seconds. The method was validated experimentally, and the variation of current with glucose concentration was fitted with a linear function described by the equation *Δj = a* + *b [glucose],* where *a* = (0.015 ± 0.005) µA cm⁻² and *b* = (0.58 ± 0.031) µA cm⁻² mM⁻¹, for which R² = 0.991 and linearity range below 0.25 mM were obtained. The limit of detection (*LOD*) was evaluated as *LOD = 3 SD*/*b,* where *SD* is the standard deviation and b is the slope of the fitting curve. The limit of quantification (*LOQ*) was determined as *LOQ = 10 SD*/*b.* The limit of detection of (25 ± 1,325) µM, the sensitivity of (0,58 ± 0,031) µA cm-2 mM-1 and the limit of quantification of (83 ± 4,399) µM were obtained.

**Example 4.** The detection and quantification of nucleic acids by voltammetric techniques can be performed in the presence of redox probes such as methylene blue. Thus, in a first step, a volume of 40 µL of a 500 µM methylene blue solution in phosphate buffer is introduced into the fluidic channel and the voltammogram is recorded using a pulsed technique (square-wave or differential pulse voltammetry). Subsequently, a volume of 4 µL of a single- or double-stranded nucleic acid solution is injected into the channel and allowed 3 minutes for diffusion and interaction between the two compounds, and afterwards the voltammogram is recorded. The process can be repeated several times, with different concentrations of nucleic acid solutions, observing the decrease in the current specific to the methylene blue reduction reaction, due to the decrease in the number of free molecules in solution. The described method was experimentally validated using double-stranded DNA (*dsDNA*) solutions, and the variation of the current density as a function of nucleic acids concentration was fitted with a line described by the equation *Δj* = *a* + *b log₂([dsDNA]*/ *µg mL⁻¹),* where *a* = (24.94 ± 1.46) µA cm⁻² and b = (49.25 ± 1.63) µA cm⁻² (µg mL⁻¹)⁻¹, for which R² = 0.996 was obtained, in the linearity range from 0.5 to 32.0 µg mL⁻¹. The limit of detection (*LOD*) was evaluated as *LOD=3 SD*/*b,* where *SD* is the standard deviation and b is the slope of the fitting curve. The limit of quantification (*LOQ*) was determined as *LOQ* = *10 SD*/*b*. The limit of detection of (0.147 ± 0.005) µg mL⁻¹, the sensitivity of (49.25 ± 1.63) µA cm⁻²(µg mL⁻¹)⁻¹ and the limit of quantification of (0.491 ± 0.016) µg mL⁻¹ were obtained.

**Example 5.** The detection and quantification of nucleic acids can be performed by electrochemical impedance spectroscopy. Firstly, a volume of 40 µL of phosphate buffer is introduced into the fluidic channel, a potential of +0.20 V (vs. Ag) and a sinusoidal perturbing signal with an amplitude of 10 mV and frequencies between 10 and 100 Hz are applied. The time evolution of the impedance is evaluated for each frequency and, after stabilization of the signal, periodic injections of solutions containing nucleic acids are made every 200 seconds. Finally, the increase of the imaginary component of the impedance is evaluated as a function of solution concentration. The described method was experimentally validated using double-stranded DNA (*dsDNA*) solutions, for which the variation of the imaginary component of impedance as a function of nucleic acid concentration was fitted with a straight line described by the equation - *ΔZ*" *= a* + *b log₂([dsDNA]*/ *µg mL⁻¹),* where *a* = (18.72 ± 0.69) Q and b = (17.15 ± 0.69) Ω cm² (µg mL⁻¹)⁻¹, for which R² = 0.994 was obtained, in the linearity range from 0.5 to 64 µg mL⁻¹. The limit of detection (*LOD*) was evaluated as *LOD = 3 SD*/*b,* where *SD* is the standard deviation and b is the slope of the fitting curve. The limit of quantification (*LOQ*) was determined as *LOQ = 10 SD*/*b.* The limit of detection of (0,25 ± 0,01) µg mL⁻¹, the sensitivity of (17,15 ± 0,69) Ω cm² (µg mL⁻¹)⁻¹ and the limit of quantification of (0,85 ± 0,034) µg mL⁻¹ were obtained.

## Claims

1. An electrochemical device for the detection of an analyte comprising:
- a first polymeric layer comprising a first electrode (6),
- a second polymeric layer comprising a second electrode (8),
- a third layer for the placement of an analyte between the two polymeric layers,
wherein the first and second polymeric layers are made of polymeric meshes (5), composed of polymeric fibers, and
wherein the third layer comprises a fibrillar hydrophilic substrate (1) having at least one hydrophilic channel therein, comprising an analyte insertion zone (3) and at least one hydrophilic region bounded by a fluid impermeable barrier (2) and wherein the hydrophilic region is in contact with the two electrodes (6,8).

2. The electrochemical device according to claim 1, wherein at least one of the first and second polymeric layers comprises at least one reagent immobilization zone located on the electrode surface in the contact area with the hydrophilic region.

3. The electrochemical device according to claim 1, wherein the third layer for placement of an analyte, located between the two polymeric layers, comprises at least one area for the immobilization of a reagent.

4. The electrochemical device according to claims 1 to 3, wherein the fibrillar hydrophilic substrate (1) is selected from: cellulose fiber paper, cotton fiber paper, glass fiber paper, filter paper, chromatography paper, commercial paper for printing or writing, cotton fabric, polyester fabric, polyamide fabric, polyacrylamide hydrogels.

5. The electrochemical device according to claims 1 to 4, wherein at least one of the first electrode and the second electrode (6,8) is made of a metal selected from gold, silver, platinum, palladium or metal oxides.

6. The electrochemical device according to claim 2 or 3, wherein the immobilized reagent is selected from: proteins, nucleic acids, organic molecules, metal complexes, salts, acids, bases.

7. A method for fabricating an electrochemical device according to claim 1, comprising the steps:
Step 1. fabricating a hydrophilic channel in a fibrillar hydrophilic substrate, comprising the steps:
i. providing the fibrillar hydrophilic substrate (1);
ii. designing the hydrophilic channel geometry;
iii. imprinting the hydrophilic channel onto the hydrophilic substrate by depositing a layer of fluid impermeable material (2) on the hydrophilic substrate so as to define a hydrophilic region;
iv. heat treating the hydrophilic substrate onto which the hydrophilic channel has been imprinted,
Step 2. The fabrication of electrodes on a first polymeric layer and a second polymeric layer,
comprising the steps of:
i. producing the polymer fibers by spinning a polymer solution;
ii. fabricating a polymeric mesh (5) by collecting on a frame the polymeric fibers resulting from the previous step;
iii. measuring the optical transmittance of the polymer meshes by UV-Vis spectrophotometry;
iv. fabricating the electrodes by metallizing at least a part of the surface of each polymeric mesh,
Step 3. Assembling the electrochemical device by placing the fibrillar hydrophilic substrate (1) containing the hydrophilic channel, resulting from step 1, between the first and second polymeric layers (5), comprising the first and second electrodes, respectively, resulting from step 2, and heating the whole assembly at temperatures between 50 and 150 °C for 2-15 minutes.

8. A procedure for fabricating an electrochemical device according to claim 7, wherein in step 2 of fabricating the electrodes on a first layer and a second polymeric layer, the spinning method is selected from electro spinning, rotary-jet spinning or dry spinning.

9. A procedure for fabricating an electrochemical device according to claim 7, wherein in step 2 of fabricating the electrodes on a first layer and a second polymeric layer, the spinning method is the electrospinning of a solution selected from: poly(methyl methacrylate), nylon, ε-polycaprolactone, polylactic acid, cellulose acetate, polyethylene oxide, polyaniline, polypyrrole, poly(3,4-ethylenedioxythiophene), polyvinyl chloride, polyurethane, polyvinylidene fluoride, or combinations thereof.

10. A method for fabricating an electrochemical device according to claim 7, wherein in step 2 of fabricating the electrodes on a first and a second polymeric layer, the metallization of a part of the surface of each polymeric mesh is carried out by thermal evaporation or magnetron sputtering, with a layer thickness between 5 and 300 nm, preferably between 50 and 150 nm, using a metal target.

11. A procedure for fabricating an electrochemical device according to claims 8 to 10, wherein in step 1 of fabricating the hydrophilic channel in the fibrillar hydrophilic substrate, the imprinting of the hydrophilic channel onto the hydrophilic substrate is performed using wax filaments or low melting point polymers, followed by heat treatment for 20 to 30 minutes at temperatures between 120 to 220 °C, preferably between 150 to 190 °C.

12. A method for the electrochemical detection of an analyte, present in a fluidic sample, using the electrochemical device according to claim 1, comprising the steps of:
a. depositing on one of the layers a solution containing a reagent;
b. depositing a fluidic sample containing the analyte into the hydrophilic channel of the fibrillar hydrophilic substrate until a fluidic contact between the sample, the reagent and the electrodes is achieved; and
c. measuring an electrochemical signal using the electrodes.

13. A method for electrochemically detecting an analyte according to claim 12, wherein the fluidic sample is selected from: plasma, serum, blood, saliva, saliva, sweat, urine, body fluids, water, contaminated soils.

14. A method for the electrochemical detection of an analyte according to claim 12, wherein the reagent is methylene blue and the analyte is a nucleic acid and the detection is accomplished by measuring the reduction current of the methylene blue after the interaction with the nucleic acids.

15. A method for the electrochemical detection of an analyte according to claim 12, wherein the analyte is a nucleic acid and the detection is accomplished by monitoring the evolution of the electrochemical impedance.
